# EUROPEAN PATENT APPLICATION

(11) **EP 4 632 057 A1**
(43) Date of publication of application: **15.10.2025**
(21) Application number: 23900364.3
(22) Date of filing: 06.11.2023
(51) Int. Cl.: C12M 1/00, C12N 1/00

(54) **CELL CULTURE CONTROL SYSTEM, CELL CULTURE CONTROL METHOD, AND CELL CULTURE CONTROL PROGRAM**

(30) Priority: 05.12.2022 JP 2022194537
(71) Applicant: Yokogawa Electric Corporation, Tokyo 180-8750 (JP)
(72) Inventor: SASAKI, Hirokazu, Musashino-shi, Tokyo 180-8750 (JP); ABE, Koki, Musashino-shi, Tokyo 180-8750 (JP); TSURUGAI, Sadakazu, Musashino-shi, Tokyo 180-8750 (JP); KINAMI, Shouta, Musashino-shi, Tokyo 180-8750 (JP)
(74) Representative: Winter, Brandl - Partnerschaft mbB
(86) International application number: PCT/JP2023/039874
(87) International publication number: WO 2024/122242

(57) **Abstract**

A cell culture control system (100) includes: a bioreactor (3) that cultures a cell; an analyzer (4) that analyzes a culture state of the cell; a bioreactor control device (1), and an advice generation server (2). The advice generation server (2) includes an advice generation unit (23) that generates culture control information based on culture data obtained from the bioreactor (3) and the analyzer (4). The bioreactor control device (1) includes: a control unit (14) that controls the bioreactor (3); and an advice processing unit (151) that acquires culture control information from the advice generation server (2) and instructs the control unit (14) to perform, on the bioreactor (3), control for allowing the cell to grow and extending a lifespan of the cell in accordance with the culture control information.

## Description

### Field

The present invention relates to a cell culture control system, a cell culture control method, and a cell culture control program.

### Background

Bioreactors are devices that synthesize and decompose substances by using biocatalysts such as microorganisms and enzymes, and are also called biological reaction devices. The bioreactors have been researched and developed as core technology together with recombinant DNA technology and cell fusion technology in various industrial fields such as food, clothing, diagnostic service, agriculture and livestock industry, fishery industry, chemical products, resource energy, bioelectronics, and environmental purification.

In operations of such bioreactors, as in other fields, the need for operation support has rapidly increased in recent years from the viewpoints of reducing individual differences in manual operation, cutting human errors, and expanding operation ranges. The following has been proposed as a procedure of using an operation support tool of a bioreactor. For example, the operation support tool is adjusted based on a predetermined procedure manual. Then, an operator, who actually operates the bioreactor, operates the bioreactor with reference to operation information provided from the operation support tool.

Furthermore, a technique of detecting a change in the total amount of proteins contained in a culture medium while cells grow in the bioreactor has been proposed as a technique of supporting the operation of the bioreactor (Patent Literature 1).

### Citation List

### Patent Literature

Patent Literature 1: JP 2021-48773 A

### Non Patent Literature

Non Patent Literature 1: Yokogawa technical report Vol. 43 No. 3 (1999) Exapilot Operation Efficiency Improvement Support Package

### Summary

### Technical Problem

When cell culture is operated, however, an operation change in accordance with a culture situation may be preferable since a control target is a living thing. In this regard, in a conventional technique, an operation support tool is adjusted in accordance with a predetermined procedure manual, so that the operation change in accordance with a culture situation is difficult, and a bioreactor has difficulty in appropriately performing cell culture.

In one aspect of the present invention, a bioreactor is automatically controlled in accordance with culture control information generated based on culture data obtained from the bioreactor and an analyzer, which enables the bioreactor to appropriately perform cell culture. Solution to Problem

According to one aspect, a cell culture control system includes a bioreactor that cultures a cell, an analyzer that analyzes a culture state of the cell, a cell culture control device, and a culture control information generation device, wherein the culture control information generation device includes a culture control information generation unit that generates culture control information based on culture data obtained from the bioreactor and the analyzer, and the cell culture control device includes a control unit that controls the bioreactor, and a culture control information processing unit that acquires the culture control information from the culture control information generation device, and instructs the control unit to perform, on the bioreactor, control of allowing the cell to grow or extending a lifespan of the cell in accordance with the culture control information.

### Advantageous Effects of Invention

According to the present invention, a bioreactor can appropriately perform cell culture.

### Brief Description of Drawings

FIG. 1 is a block diagram of a cell culture control system according to an embodiment.
FIG. 2 illustrates transitions of glucose and cells under nutrient depletion prevention control.
FIG. 3 illustrates transitions of input amounts of glucose and a feed agent under nutrient input ratio control.
FIG. 4 illustrates an example of advice data.
FIG. 5 is a flowchart of operation support processing performed by a bioreactor control device according to the example.
FIG. 6 is a flowchart of advice acceptance processing.
FIG. 7 is a flowchart of bioreactor control processing in accordance with advice.
FIG. 8 is a block diagram of a cell culture control system using edge computing.
FIG. 9 illustrates an example of the hardware configuration of the bioreactor control device.
FIG. 10 illustrates another example of the hardware configuration of the bioreactor control device. Description of Embodiments

An embodiment of a cell culture control system, a cell culture control method, and a cell culture control program will be described below with reference to the drawings. Note that the same elements are denoted by the same reference signs, and redundant description will be appropriately omitted. Furthermore, the embodiment can be appropriately combined within a range without inconsistency.

### (Overall Configuration)

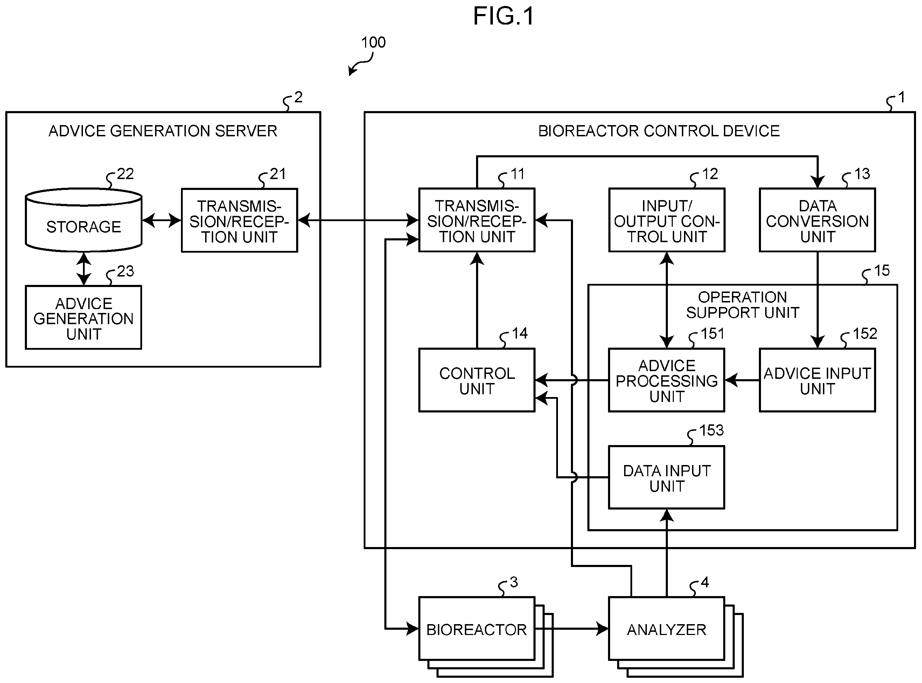

FIG.1 is a block diagram of the cell culture control system according to the embodiment. A cell culture control system 100 includes a bioreactor control device 1, an advice generation server 2, a bioreactor 3, and an analyzer 4. The cell culture control system 100 generates advice on an appropriate operation from the state of cell culture in the bioreactor 3, presents the advice to an operator, and controls the operation of the bioreactor 3 in accordance with the advice, which is culture control information generated with the permission of the operator.

The bioreactor 3 is a device for culturing cells. The purposes of cell culture performed by the bioreactor 3 is as follows. One is to increase the number of cells to manage and obtain the cells themselves. The other is to obtain products such as antibodies from cells. In order to obtain more products, increasing the number of cells and making the cells live longer is important.

Although not illustrated, various sensors, such as a temperature sensor, a pH sensor, and a sensor for measuring a lactic acid value, are attached to the bioreactor 3. The bioreactor 3 acquires sensor information by using the various sensors. Furthermore, a mechanism for measuring culture solution information such as the weight and volume of a culture solution is attached to the bioreactor 3. Moreover, the bioreactor 3 can calculate predicted values of cell density, glucose concentration, and a lactic acid value by using NIR and Raman spectroscopy. The sensor information and the culture solution information include a viable cell density predicted value, a glucose concentration predicted value, a lactic acid value predicted value, a dissolved oxygen amount, a pH, a culture tank temperature, a culture solution weight, and a culture solution volume. The sensor information and the culture solution information obtained from the bioreactor 3 may also be referred to as in-line data. The bioreactor 3 notifies the bioreactor control device 1 of the sensor information and the culture solution information obtained by the various sensors. One bioreactor 3 or a plurality of bioreactors 3 may be provided.

The analyzer 4 measures various types of culture state information indicating the state of a culture solution, such as the concentration of glucose and the density of cells, by acquiring a culture solution of the bioreactor 3 and analyzing the acquired culture solution. Examples of the culture state information include cell density, viable cell density, dead cell density, a cell viability, cell diameter, cell roundness, glucose concentration, glutamine, glutamic acid, a lactic acid value, ammonium, IgG, LDH, sodium, potassium, calcium, pH, PO2, PCO2, and Osm. The analyzer 4 may be an offline analyzer or an at-line analyzer. In the offline analyzer, the operator manually acquires a culture solution from the bioreactor 3, and inputs the culture solution to the analyzer 4. In the at-line analyzer, a culture solution is automatically acquired from the bioreactor 3. When being an at-line analyzer, the analyzer 4 may transmit the measured culture state information to the bioreactor 3. Furthermore, one analyzer 4 or a plurality of analyzers 4 may be provided. The culture state information obtained by the offline analyzer or the at-line analyzer may be referred to as offline data or at-line data, respectively.

A predicted value of a culture state is obtained from the bioreactor 3. In contrast, the analyzer 4 measures the culture state information by analyzing an actual culture solution. The cell culture control system 100 can thus obtain the culture state information with higher accuracy by using a measured value from the analyzer 4. The analyzer 4 outputs the measured culture state information to the bioreactor control device 1.

The bioreactor control device 1 is connected to the advice generation server 2 via a network. Furthermore, the bioreactor control device 1 is connected to the bioreactor 3. The bioreactor control device 1 acquires the sensor information and the culture solution information from the bioreactor 3. Furthermore, the bioreactor control device 1 acquires, from the analyzer 4, the culture state information obtained by the measurement performed by the analyzer 4. Furthermore, the bioreactor control device 1 acquires advice data generated by the advice generation server 2 based on culture data obtained by combining the sensor information, the culture solution information, and the culture state information. Then, the bioreactor control device 1 confirms with the operator whether or not to accept advice included in the acquired advice data. When the operator accepts the advice, the bioreactor control device 1 controls the operation of the bioreactor 3 in accordance with the advice. The bioreactor control device 1 is an example of a "cell culture control device".

The advice generation server 2 is connected to the bioreactor control device 1 via the network. The advice generation server 2 acquires culture data from the bioreactor control device 1, and generates advice data including advice on the operation of the bioreactor 3 in accordance with the acquired culture data. Then, the advice generation server 2 transmits the generated advice data to the bioreactor control device 1. The advice generation server 2 may be a device of an in-house system managed on the premises or a device existing on a cloud. The advice generation server 2 is an example of a "culture control information generation device".

### (Control Device)

Next, details of the bioreactor control device 1 will be described with reference to FIG. 1. As illustrated in FIG. 1, the bioreactor control device 1 includes a transmission/reception unit 11, an input/output control unit 12, a data conversion unit 13, a control unit 14, and an operation support unit 15.

The transmission/reception unit 11 controls transmission and reception of data to and from the advice generation server 2. The transmission/reception unit 11 receives inputs of culture data of the bioreactor 3 including the sensor information, the culture solution information, and the culture state information from the bioreactor 3 and the analyzer 4. Then, the transmission/reception unit 11 transmits the acquired culture data of the bioreactor 3 to the advice generation server 2. Furthermore, the transmission/reception unit 11 receives advice data generated based on the transmitted culture data from the advice generation server 2. Then, the transmission/reception unit 11 outputs the acquired advice data to the data conversion unit 13.

As described above, for example, the transmission/reception unit 11 relays communication between the data conversion unit 13 and the control unit 14 and the advice generation server 2. Note, however, that the transmission/reception unit 11 may be omitted below for easy understanding, and the data conversion unit 13 and the control unit 14 may be described as directly communicating with the advice generation server 2.

The input/output control unit 12 includes a display device such as a monitor and an input device such as a mouse and a keyboard. The input/output control unit 12 controls data transfer to and from the operator via the display device, the input device, or the like. For example, the input/output control unit 12 causes the display device to display an advice acceptance selection screen for causing the operator to select whether or not to accept advice included in advice data provided from the operation support unit 15, and causes the operator to select whether or not to accept the advice. Then, the input/output control unit 12 acquires, from the input device, information on whether or not to accept the advice selected by the operator by using the advice acceptance selection screen displayed by the display device. Thereafter, the input/output control unit 12 outputs information on whether or not to accept the advice to the operation support unit 15.

The data conversion unit 13 receives, from the transmission/reception unit 11, input of the advice data transmitted from the advice generation server 2. Then, the data conversion unit 13 converts the advice data transmitted from the advice generation server 2 into a format that can be processed by the operation support unit 15. For example, the data conversion unit 13 receives advice data in a JSON format from the advice generation server 2 as data input, and converts the advice data into data in an Excel format. Thereafter, the data conversion unit 13 outputs the advice data converted into a format that can be processed by the operation support unit 15 to the operation support unit 15.

The control unit 14 collects culture data of the bioreactor 3, and controls the operation of the bioreactor 3. For example, the control unit 14 collects the sensor information and the culture solution information measured in the bioreactor 3. Furthermore, the control unit 14 collects the culture state information measured by the analyzer 4. The culture data includes, for example, in-line data and offline/at-line data.

In the example, the control unit 14 acquires the culture state information from a data input unit 153 of the operation support unit 15. Note, however, that, when the analyzer 4 is an at-line analyzer, the control unit 14 may directly acquire the culture state information from the analyzer 4. Then, the control unit 14 combines the sensor information, the culture solution information, and the culture state information, which have been collected, and transmits these pieces of information to the advice generation server 2 via the transmission/reception unit 11 as culture data representing the state of the bioreactor 3. That is, the control unit 14 collects the culture data from the bioreactor 3 and the analyzer 4, and transmits the culture data to the culture control information generation device.

Furthermore, the control unit 14 receives an instruction to control the bioreactor 3 in accordance with the advice included in the advice data from the operation support unit 15. Then, when the culture state information is used for control, the control unit 14 acquires the culture state information from the operation support unit 15 together with the instruction to control the bioreactor 3. Then, the control unit 14 controls the operation of the bioreactor 3 in accordance with the instruction. Examples of control information sent by the control unit 14 to the bioreactor 3 include a control value of a stirrer, a heater, or a sparger (e.g., air, oxygen, carbon dioxide, and nitrogen), a glucose input amount, a feed agent input amount, and an alkaline solution input amount.

For example, the control unit 14 receives an instruction to input a predetermined amount of glucose from the operation support unit 15 together with input time information. In the case, when the designated input time is reached, the control unit 14 inputs the designated amount of glucose to the bioreactor 3. This can prompt growth of cells in the bioreactor 3 to extend the lifespans of the cells and increase the number of cells. Furthermore, when an acidified culture solution have a negative effect on target cells, the control unit 14 receives an instruction to input a predetermined amount of alkaline solution from the operation support unit 15 together with the input time information. In the case, when the designated input time is reached, the control unit 14 inputs the designated amount of alkaline solution to the bioreactor 3. This can improve a culture environment in the bioreactor 3 to extend the lifespans of the cells and increase the number of cells.

The operation support unit 15 supports the operator in operating the bioreactor 3 based on the advice data generated by the advice generation server 2. The operation support unit 15 includes an advice processing unit 151, an advice input unit 152, and the data input unit 153.

The advice input unit 152 receives, from the data conversion unit 13, input of advice data converted into a format that can be processed by the device itself. Then, the advice input unit 152 outputs the acquired advice data to the advice processing unit 151.

The advice processing unit 151 receives, from the advice input unit 152, input of the advice data generated by the advice generation server 2. Next, the advice processing unit 151 extracts and holds an advice creation time added to the advice data. Thereafter, the advice processing unit 151 executes advice acceptance processing to be described below for each piece of advice data.

The advice processing unit 151 determines whether or not there is new advice among pieces of advice included in the held advice data by using the advice creation time. Here, the advice processing unit 151 may determine, each time the advice input unit 152 inputs advice data, whether or not the advice data contains new advice, or may periodically determine whether or not there is new advice in advice included in the accumulated advice data.

When there is new advice, the advice processing unit 151 instructs the input/output control unit 12 to display an advice selection screen for displaying whether or not to accept the new advice in a selectable manner, and causes the display device to display the advice selection screen. The advice processing unit 151 thereby confirms with the operator whether or not to accept the advice by using the advice selection screen.

When the operator makes a notification of acceptance of the advice, the advice processing unit 151 holds the advice data including the new advice permitted to be accepted as accepted advice data. In contrast, when the operator makes a notification of non-acceptance of the advice, the advice processing unit 151 ends acceptance processing for the advice.

Furthermore, the advice processing unit 151 periodically determines whether or not there is accepted advice data in the held advice data asynchronously with the acceptance processing for the advice. When there is accepted advice data, the advice processing unit 151 acquires an execution time in the nearest future among execution times of unprocessed accepted advice data. Then, the advice processing unit 151 determines whether or not the current time exceeds the acquired execution time. When the current time does not exceed the acquired execution time, the advice processing unit 151 waits until the acquired execution time.

In contrast, when the current time exceeds the acquired execution time, the advice processing unit 151 instructs the control unit 14 to control the bioreactor 3 in accordance with advice included in the advice data. In the case, when culture state information from the analyzer 4 is used in the control in accordance with the advice, the advice processing unit 151 instructs the data input unit 153 to input the culture state information to be used to the control unit 14.

The advice processing unit 151 instructs the control unit 14 to control the bioreactor 3 in accordance with the advice included in all the unprocessed accepted advice data. When there is no unprocessed accepted advice data, the advice processing unit 151 waits until new accepted advice data is generated.

Here, control in accordance with advice will be described. The bioreactor control device 1 controls the bioreactor 3 to increase the number of cells and make the cells live longer. That is, control in accordance with advice given from the advice processing unit 151 to the control unit 14 is also control to increase the number of cells and make the cells live longer. Examples of the control for the purposes include nutrient depletion prevention control and nutrient input ratio control. Under the nutrient depletion prevention control, control is performed to prevent depletion of nutrients in a growth phase of cells. Under the nutrient input ratio control, a ratio of inputs of various nutrients is appropriately controlled in a growth phase, a stationary phase, and a death phase.

For example, under the nutrient depletion prevention control, the advice processing unit 151 instructs the control unit 14 to perform control in accordance with advice on a glucose input amount based on a monitoring result such as a viable cell density and a glucose concentration.

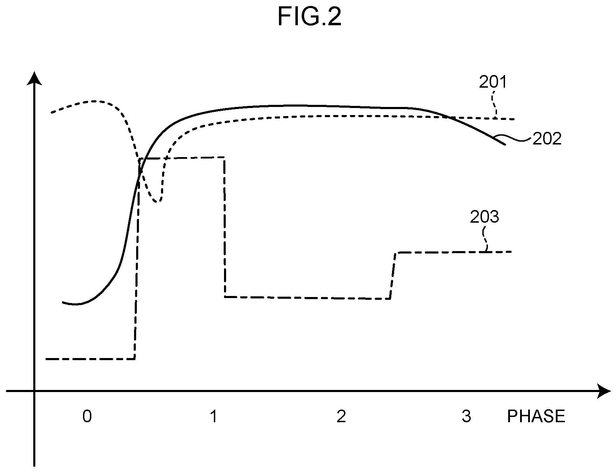

FIG. 2 illustrates transitions of glucose and cells under the nutrient depletion prevention control. A graph 201 represents changes in glucose concentration. A graph 202 represents changes in viable cell density. A graph 203 represents a glucose input amount. In FIG. 2, the vertical axis represents the glucose concentration, the viable cell density, or the glucose input amount in accordance with the graphs 201 to 203, and the horizontal axis represents a phase. In FIG. 2, a phase of 0 represents a delay phase, a phase of 1 represents a growth phase, a phase of 2 represents a stationary phase, and a phase of 3 represents a death phase. For example, when the viable cell density rapidly increases and the glucose concentration rapidly decreases, the advice processing unit 151 instructs the control unit 14 to perform control in accordance with advice for increasing the glucose input amount in order to increase the glucose concentration and make the cells live longer. That is, the advice processing unit 151 causes the control unit 14 to control the bioreactor 3 so as to increase culture data collection frequency in the growth phase of cells as compared with those in the stationary phase and the death phase.

Here, since the glucose concentration and the cell density may change in the growth phase of cells more rapidly than those in other phases, the frequency of data collection performed by the control unit 14 may be increased. For example, in order to increase the data collection frequency, the control unit 14 may utilize in-line data that can increase the collection frequency. Furthermore, when the data collection frequency is increased, an advice generation unit 23 of the advice generation server 2 to be described later may reduce an analysis load by narrowing the types of data to be analyzed to increase a speed.

Furthermore, for example, under the nutrient input ratio control, the advice processing unit 151 gives an instruction to perform control in accordance with advice of increasing a feed agent input ratio toward the death phase based on the growth phase, the stationary phase, and the death phase determined by using culture data such as viable cell density.

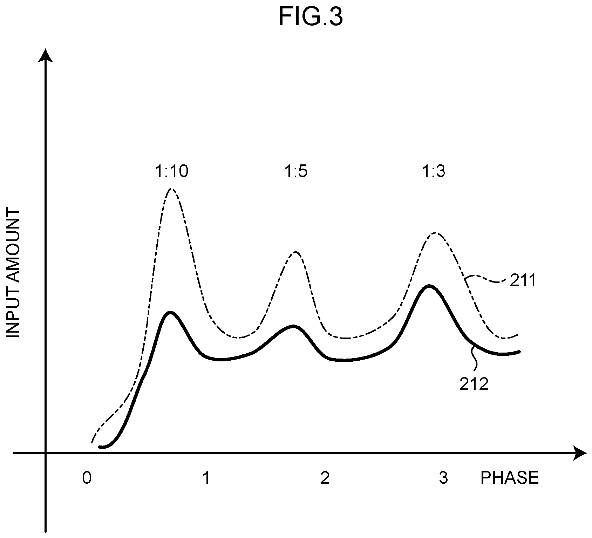

FIG.3 illustrates transitions of input amounts of glucose and a feed agent under the nutrient input ratio control. A graph 211 represents changes in a glucose input amount. A graph 212 represents changes in a feed agent input amount. In FIG. 2, the vertical axis represents an input amount, and the horizontal axis represents Ph. For example, when the slope of the viable cell density decreases, the advice processing unit 151 instructs the control unit 14 to perform control to increase the ratio of the feed agent input amount based on the determination that shift has been performed from the growth phase to the stationary phase. Furthermore, the advice processing unit 151 instructs the control unit 14 to perform control to increase the ratio of the feed agent input amount based on the determination that shift has been performed from the stationary phase to the death phase in accordance with the slope of the cell density and time.

The data input unit 153 acquires culture state information obtained by measurement performed by the analyzer 4. The data input unit 153 may automatically acquire the culture state information from the analyzer 4, or may acquire the culture state information by manual input of the operator. Then, the data input unit 153 outputs the acquired culture state information to the control unit 14.

Furthermore, the data input unit 153 receives, from the advice processing unit 151, an instruction to input the culture state information to be used to the control unit 14. Then, the data input unit 153 acquires the culture state information to be used designated by the data input unit 153 from the analyzer 4. Thereafter, the data input unit 153 outputs the culture state information acquired from the analyzer 4 to the control unit 14.

### (Server)

Next, details of the advice generation server 2 will be described with reference to FIG. 1. As illustrated in FIG. 1, the advice generation server 2 includes a transmission/reception unit 21, a storage 22, and the advice generation unit 23.

The transmission/reception unit 21 controls transmission and reception of data to and from the bioreactor control device 1. The transmission/reception unit 21 receives culture data, including the sensor information, the culture solution information, and the culture state information, of the bioreactor 3 from the transmission/reception unit 11 of the bioreactor control device 1. Then, the transmission/reception unit 21 stores the acquired culture data of the bioreactor 3 in the storage 22.

Furthermore, the transmission/reception unit 21 acquires advice data including advice and a creation time of each advice stored in the storage 22. Then, the transmission/reception unit 21 transmits the advice data to the transmission/reception unit 11 of the bioreactor control device 1.

The storage 22 stores the culture data of the bioreactor 3 received by the transmission/reception unit 21 from the bioreactor control device 1. Furthermore, the storage 22 stores advice data including advice generated by the advice generation unit 23 and a creation time of the advice.

The advice generation unit 23 acquires the culture data of the bioreactor 3 stored in the storage 22. Then, the advice generation unit 23 generates advice data on the operation of the bioreactor 3 based on the culture data. More specifically, the advice generation unit 23 creates advice data written in a JSON format or the like indicating advice on the operation.

For example, the advice generation unit 23 may generate the advice data by determining advice in accordance with culture data input by using a simple condition determination. In addition, the advice generation unit 23 may generate the advice data by determining advice in accordance with culture data input by using artificial intelligence (AI).

For example, the advice generation unit 23 generates advice data on processing for allowing cells in the bioreactor 3 to grow and extending the lifespans of the cells. More specifically, the advice generation unit 23 generates advice data on what hour and what minute how many milliliters of which solution is input. In the case, the advice generation unit 23 may add, for example, the reason for an operation to be executed to the advice data. When the data collection frequency increases in the growth phase of cells, the advice generation unit 23 may reduce an analysis load by narrowing the types of data to be analyzed to increase the speed of analysis processing.

For example, the advice generation unit 23 acquires viable cell density and glucose concentration from the culture data. When determining that too small an amount of glucose for cells to be cultured inhibits growth of the cells, the advice generation unit 23 generates advice data of designating the time and amount of input of glucose. Furthermore, the advice generation unit 23 acquires a lactic acid value included in the sensor information. When determining that an acid culture solution inhibits growth of the cells, the advice generation unit 23 generates advice data of designating the time and amount of input of an alkaline solution.

In addition, the advice generation unit 23 determines the growth phase, the stationary phase, and the death phase based on culture data such as viable cell density, and generates advice data of increasing the feed agent input ratio toward the death phase. For example, the advice generation unit 23 detects a decrease in an increasing trend of viable cell density, and determines shift of cells from the growth phase to the stationary phase. Furthermore, the advice generation unit 23 determines shift from the stationary phase to the death phase based on viable cell density and elapsed time.

Here, the advice data is an example of the "culture control information". The advice generation unit 23 is an example of a "culture control information generation unit". That is, the advice generation unit 23 determines the growth phase, the stationary phase, and the death phase of cells based on the culture data, and generates the culture control information so as to increase the feed agent input ratio in the order of the growth phase, the stationary phase, and the death phase.

Then, the advice generation unit 23 adds the creation time of each piece of advice to the generated advice data, and stores the advice data in the storage 22.

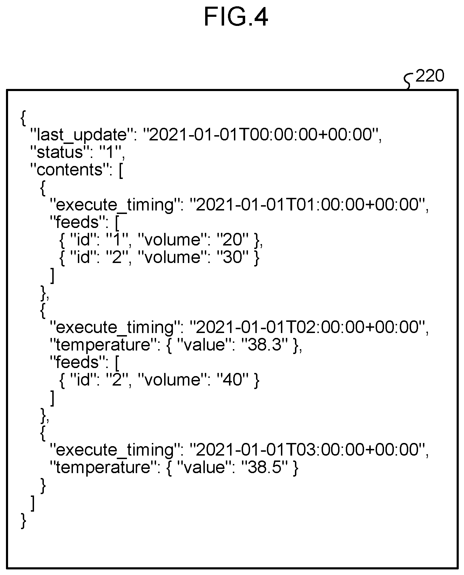

FIG. 4 illustrates an example of the advice data. The advice generation unit 23 generates advice data 220 written in the JSON format as illustrated in FIG.4, for example. In the advice data 220, "last_update" is advice creation or transmission time, "status" is advice priority, urgency, or the like, "contents" is operation information of the bioreactor 3, and "execute_timing" is advice execution time. Moreover, "feeds" is control information of various pumps such as a pump ID, an input amount, and a quantitative feeding time, and "temperature" is control information of a heater. The advice data may further include sparger control information for various gases and information on stirring control and a culture solution sampling instruction. The advice generation unit 23 can designate execution time, processing to be executed, and the like by using the advice data 220 and the like.

### (Flow of Operation Support Processing)

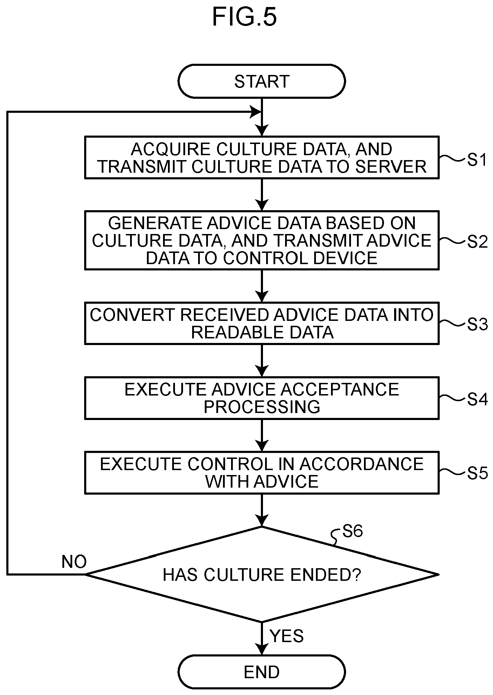

FIG. 5 is a flowchart of operation support processing performed by the bioreactor control device according to the example. Next, a flow of the operation support processing performed on the bioreactor 3 by the operator by the bioreactor control device 1 according to the example will be described with reference to FIG. 5.

The control unit 14 collects the sensor information and culture solution information measured in the bioreactor 3. Furthermore, the control unit 14 collects the culture state information measured by the analyzer 4. Then, the control unit 14 transmits the culture data obtained by combining the sensor information, the culture solution information, and the culture state information, which have been collected, to the advice generation server 2 (Step S1).

The transmission/reception unit 21 of the advice generation server 2 receives the culture data transmitted from the control unit 14 of the bioreactor control device 1, and stores the culture data in the storage 22. The advice generation unit 23 acquires the culture data stored in the storage 22, generates advice data on the operation of the bioreactor 3 in accordance with the culture data, and stores the advice data in the storage 22. The transmission/reception unit 21 transmits advice data including advice and an advice creation time stored in the storage 22 to the bioreactor control device 1 (Step S2).

The transmission/reception unit 11 receives the advice data from the transmission/reception unit 21 of the advice generation server 2, and outputs the advice data to the data conversion unit 13. The data conversion unit 13 converts the advice data transmitted from the advice generation server 2 into a format that can be processed by the operation support unit 15 (Step S3).

The operation support unit 15 acquires the advice data converted into a format that can be processed by the data conversion unit 13. Then, the operation support unit 15 executes advice acceptance processing of confirming with the operator whether or not to accept the advice (Step S4).

Furthermore, the operation support unit 15 instructs the control unit 14 to control the operation of the bioreactor 3 in accordance with advice included in the advice data. The control unit 14 controls the operation of the bioreactor 3 in accordance with the advice given by the operation support unit 15, and operates the bioreactor 3 (Step S5).

The control unit 14 and the operation support unit 15 determine whether or not the culture of cells in the bioreactor 3 has ended (Step S6). When the culture of cells has not ended (Step S6: No), the operation support processing returns to Step S1. In contrast, when the culture of cells has ended (Step S6: Yes), the control unit 14 and the operation support unit 15 end the operation support processing.

Although the advice acceptance processing is set as Step S4, and the control of the bioreactor 3 in accordance with advice is set as Step S5 here for convenience of illustration, actually, the bioreactor control device 1 performs the advice acceptance processing and the control in accordance with advice in parallel. Furthermore, the bioreactor control device 1 may perform the processing in Steps S1 to S3 in parallel with these pieces of processing.

### (Advice Acceptance Processing)

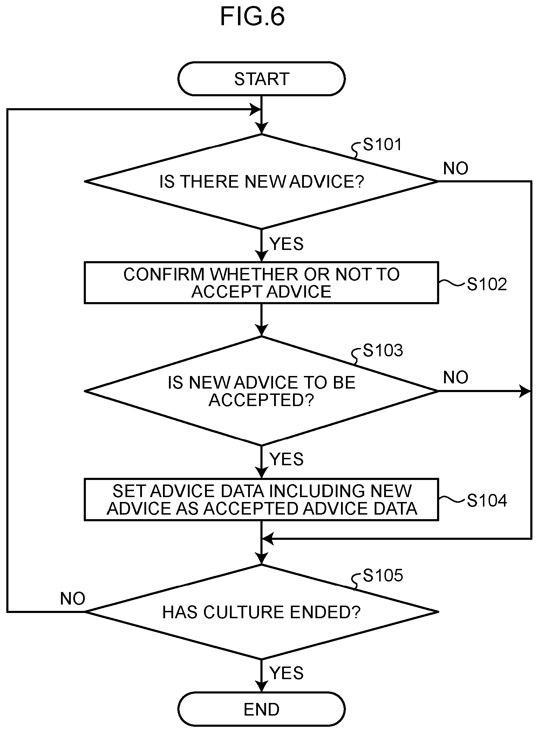

FIG. 6 is a flowchart of the advice acceptance processing. Each piece of processing of a flow in FIG. 6 is an example of processing executed in Step S4 in FIG. 5.

The advice processing unit 151 receives, from the advice input unit 152, input of the advice data generated by the advice generation server 2. Next, the advice processing unit 151 extracts and holds an advice creation time. Then, the advice processing unit 151 determines whether or not there is new advice among pieces of advice included in the held advice data (Step S101). When there is no new advice (Step S101: No), the advice acceptance processing proceeds to Step S105.

In contrast, when there is new advice (Step S101: Yes), the advice processing unit 151 outputs, to the input/output control unit 12, an advice selection screen for displaying whether or not to accept the new advice in a selectable manner, and causes the display device to display the advice selection screen. The advice processing unit 151 thereby confirms with the operator whether or not to accept the new advice by using the advice selection screen (Step S102).

The advice processing unit 151 determines whether or not to accept the new advice based on a selection result from the operator using the advice selection screen displayed on the display device (Step S103). When the new advice is not accepted (Step S103: No), the advice acceptance processing proceeds to Step S105.

In contrast, when the new advice is accepted (Step S103: Yes), the advice processing unit 151 sets advice data including the new advice as accepted advice data (Step S104).

Thereafter, the advice processing unit 151 determines whether or not the culture of cells has ended (Step S105). When the culture of cells has not ended (Step S105: No), the advice acceptance processing returns to Step S101.

In contrast, when the culture of cells has ended (Step S105: Yes), the advice processing unit 151 ends the advice acceptance processing.

### (Bioreactor Control Processing)

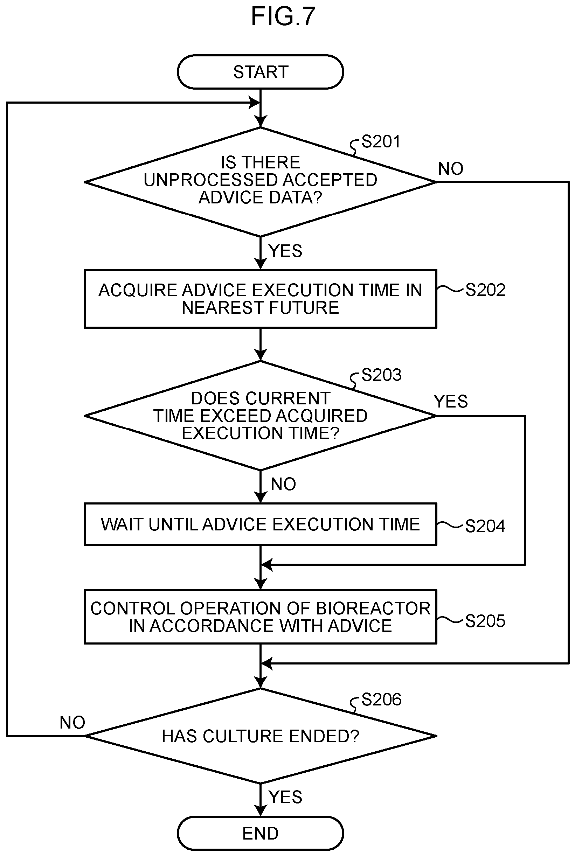

FIG. 7 is a flowchart of bioreactor control processing in accordance with advice. Each piece of processing of a flow in FIG. 7 is an example of processing executed in Step S5 in FIG. 5.

The advice processing unit 151 determines whether or not there is unprocessed accepted advice data among pieces of held advice data (Step S201). When there is no unprocessed accepted advice data (Step S201: No), the bioreactor control processing proceeds to Step S206.

In contrast, when there is unprocessed accepted advice data (Step S201: Yes), the advice processing unit 151 acquires an execution time in the nearest future among execution times of unprocessed accepted advice data (Step S202).

Next, the advice processing unit 151 determines whether or not the current time exceeds the acquired execution time (Step S203). When the current time exceeds the advice execution time (Step S203: Yes), the bioreactor control processing proceeds to Step S205.

In contrast, when the current time does not exceed the advice execution time (Step S203: No), the advice processing unit 151 waits until the advice execution time (Step S204).

Thereafter, the advice processing unit 151 instructs the control unit 14 to control the bioreactor 3 in accordance with advice included in the advice data. In the case, when a measurement result from the analyzer is used in the control in accordance with the advice, the advice processing unit 151 instructs the data input unit 153 to input information of the measurement result to be used to the control unit 14. The control unit 14 receives an instruction from the advice processing unit 151, and controls the operation of the bioreactor 3 in accordance with the advice (Step S205).

Thereafter, the advice processing unit 151 determines whether or not the culture of cells has ended (Step S206). When the culture of cells has not ended (Step S206: No), the bioreactor control processing returns to Step S201.

In contrast, when the culture of cells has ended (Step S206: Yes), the advice processing unit 151 and the control unit 14 end the bioreactor control processing.

### [Effects]

As described above, the cell culture control system 100 according to the embodiment generates advice on the operation of the bioreactor 3 for allowing cells to grow and extending the lifespans of the cells based on culture data representing the latest culture situation of the bioreactor 3. Next, the cell culture control system 100 confirms with the operator whether or not to accept the generated advice. When the operator determines to accept the advice, the cell culture control system 100 controls the operation of the bioreactor 3 in accordance with the advice.

As described above, the cell culture control system 100 monitors culture data related to cell culture from the bioreactor 3 and the analyzer 4, and proposes a method of controlling the bioreactor 3 based on the culture data. This enables an operation on the bioreactor 3 to be changed in accordance with a culture situation, and enables optimum culture regardless of the skill of the operator. Moreover, an appropriate operation can be performed at an appropriate time. The lifespans of cells cultured in the bioreactor 3 can be easily extended, and the number of cells can be easily increased. The bioreactor 3 can appropriately culture cells.

### <Variations>

The control unit 14 can be implemented by a distributed control system (DCS), supervisory control and data acquisition (SCADA), a programmable logic controller (PLC), a recorder, a controller, or the like. When the control unit 14 is implemented by the SCADA, the transmission/reception unit 11, the input/output control unit 12, the data conversion unit 13, the control unit 14, and the operation support unit 15 can be implemented in combination in one computer. Furthermore, when the control unit 14 is implemented by dedicated hardware such as a DCS, a PLC, a recorder, and a controller, the control unit 14 and the operation support unit 15 can be arranged as different devices.

The transmission/reception unit 11, the control unit 14, the data input unit 153, and the transmission/reception unit 21 can use message queueing telemetry transport (MQTT), web server/client communication (representational state transfer (REST) and application programming interface (API)), open platform communications (OPC), HART IP, distributed network protocol (DNP), PROFINET, Modbus, and the like, which serve as communication interfaces. Furthermore, the transmission/reception unit 11, the control unit 14, the data input unit 153, and the transmission/reception unit 21 can use, for example, txt data, csv data, and Excel data as data to be transferred.

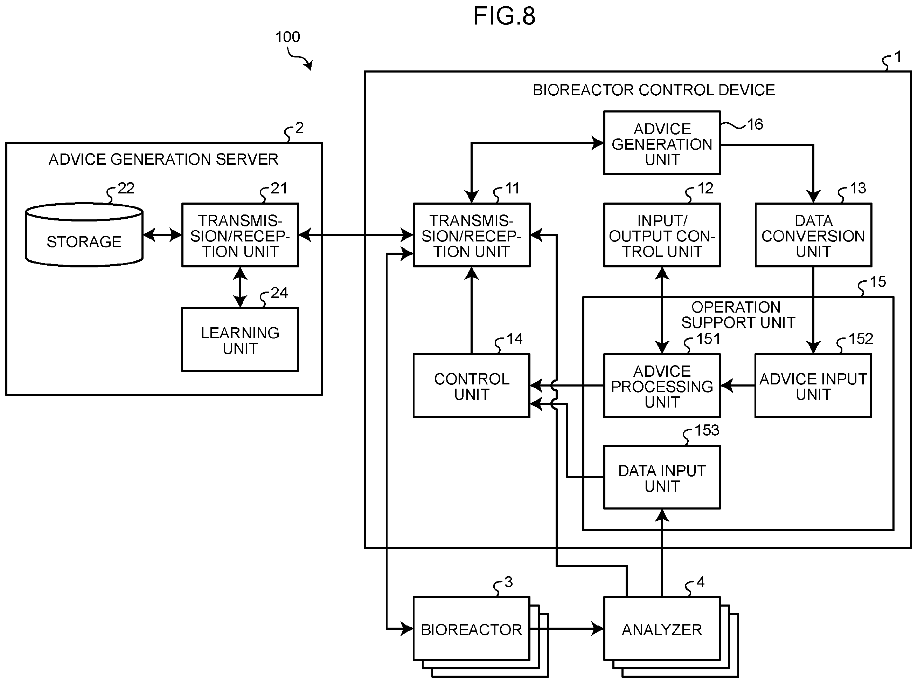

The bioreactor control device 1 may include the advice generation unit 23. FIG. 8 is a block diagram of a cell culture control system using edge computing. For example, the cell culture control system 100 can have configuration as illustrated in FIG. 8 by adopting the configuration of the edge computing.

The bioreactor control device 1 includes an advice generation unit 16 in addition to each unit described in Example 1. The advice generation unit 16 has a function of the advice generation unit 23 in the advice generation server 2 according to Example 1. In this case, the bioreactor control device 1 is not required to transmit culture data to the advice generation server 2 and receive advice from the advice generation server 2.

For example, the advice generation unit 16 can generate advice by using AI. In the case, the bioreactor control device 1 may receive learning data for performing learning of AI from the advice generation server 2, and perform learning of AI to be used by the advice generation unit 16. In this case, a learning unit 24 of the advice generation server 2 may increase the number of pieces of machine learning data. Increasing the number of pieces of machine learning data can improve the accuracy of AI.

In addition, the advice generation server 2 may hold AI, which is a digital twin of AI held by the advice generation unit 16. In the case, the advice generation server 2 may perform learning of AI, and notify the advice generation unit 16 of the bioreactor control device 1 of a hyperparameter determined by the learning.

Furthermore, although, in the example, whether or not to accept advice is confirmed with the operator in order to reflect the determination of the operator, the bioreactor control device 1 can omit the confirmation operation, and completely automate the control of the operation of the bioreactor 3.

### (System)

Information including the processing procedures, the control procedures, the specific names, and various pieces of data and parameters in the above document and the drawings can be optionally changed unless otherwise specified.

Furthermore, each component of each illustrated device is functionally conceptual, and is not necessarily required to be physically configured as illustrated. That is, a specific form of distribution and integration of each device is not limited to the illustrated form. That is, all or a part thereof can be functionally or physically distributed and integrated in any unit in accordance with various loads, use situations, and the like.

Moreover, all or any part of each processing function performed in each device can be implemented by a central processing unit (CPU) and a program analyzed and executed by the CPU, or can be implemented as hardware using a wired logic.

### [Hardware]

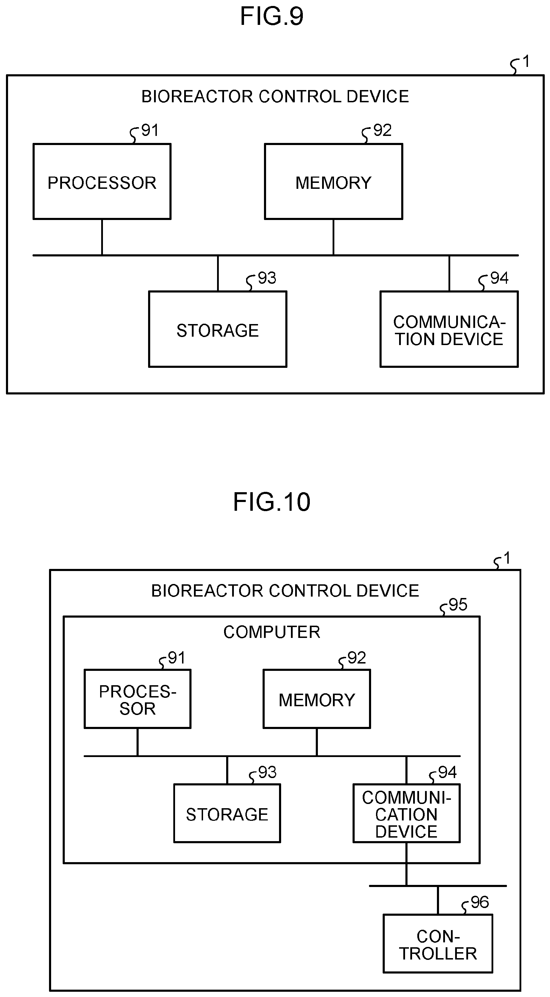

Next, an example of a hardware configuration of the bioreactor control device 1 will be described. FIG. 9 illustrates an example of the hardware configuration of the bioreactor control device. As illustrated in FIG. 9, the bioreactor control device 1 includes a processor 91, a memory 92, a storage 93, and a communication device 94. Furthermore, the processor 91 is connected to the memory 92, the storage 93, and the communication device 94 via a bus.

The communication device 94 is, for example, a network interface card, and is used for communication with other information processing devices. For example, the communication device 94 relays communication between the processor 91 and the advice generation server 2. Furthermore, the communication device 94 implements a part of the function of the transmission/reception unit 11.

The storage 93 is an auxiliary storage device. The storage 93 stores various programs including programs for implementing the functions of the transmission/reception unit 11, the input/output control unit 12, the data conversion unit 13, the control unit 14, and the operation support unit 15 illustrated in FIG. 1.

The processor 91 reads various programs stored in the storage 93, develops the programs in the memory 92, and executes the programs. This causes the processor 91 to implement the functions of the transmission/reception unit 11, the input/output control unit 12, the data conversion unit 13, the control unit 14, and the operation support unit 15.

Note, however, that, although a case where the control unit 14 and the operation support unit 15 are implemented by one computer has been described here, the control unit 14 may be a device different from a computer in which the operation support unit 15 such as a PLC, a recorder, and a controller operates. For example, the bioreactor control device 1 can be implemented by a hardware configuration in FIG. 10. FIG. 10 illustrates another example of the hardware configuration of the bioreactor control device. As illustrated in FIG. 10, the bioreactor control device 1 may include a computer 95 and a controller 96. The controller 96 is connected to the computer 95 via the communication device 94.

In this case, the controller 96 implements the function of the control unit 14. Since the control unit 14 is required to have long-term stability, it may be difficult to use a computer. The long-term stability can be secured by using the controller 96 instead. In this case, the processor 91 implements the functions of the transmission/reception unit 11, the input/output control unit 12, the data conversion unit 13, and the operation support unit 15.

As described above, the bioreactor control device 1 operates as an information processing device that executes various processing methods by reading and executing a program. Furthermore, the bioreactor control device 1 can also implement a function similar to those of the above-described embodiment by reading the above-described program from a recording medium with a medium reading device and executing the read program. Note that the program here is not limited to being executed by the bioreactor control device 1. For example, the present invention can be similarly applied even to a case where another computer or server executes a program or a case where these computer and server execute the program in cooperation.

The program can be distributed via a network such as the Internet. Furthermore, the program is recorded in a computer-readable recording medium such as a hard disk, a flexible disk (FD), a CD-ROM, a magneto-optical (MO) disk, and a digital versatile disc (DVD), and can be executed by being read from the recording medium by the computer.

Some examples of combinations of the disclosed technical features will be described below.
(1) A cell culture control system includes: a bioreactor that cultures a cell; an analyzer that analyzes a culture state of the cell; a cell culture control device; and a culture control information generation device,
   wherein the culture control information generation device includes
   a culture control information generation unit that generates culture control information based on culture data obtained from the bioreactor and the analyzer, and
   the cell culture control device includes:
      a control unit that controls the bioreactor; and
      a culture control information processing unit that acquires the culture control information from the culture control information generation device, and instructs the control unit to perform, on the bioreactor, control of allowing the cell to grow or extending a lifespan of the cell in accordance with the culture control information.
(2) The cell culture control system according to (1), wherein the culture control information processing unit confirms whether or not to accept the culture control information, and, when the culture control information is accepted, instructs the control unit to control the bioreactor in accordance with the culture control information.
(3) The cell culture control system according to (1) or (2), wherein the control unit collects the culture data from the bioreactor and the analyzer, and transmits the culture data to the culture control information generation device.
(4) The cell culture control system according to (3), wherein the culture control information processing unit causes the control unit to control the bioreactor so as to increase a frequency of collection of the culture data in a growth phase of a cell as compared with those in a stationary phase and a death phase.
(5) The cell culture control system according to (4), wherein the culture control information processing unit causes the control unit to control the bioreactor so as to increase the frequency of collection of the culture data obtained from the bioreactor that is allowed to adjust the frequency of collection.
(6) The cell culture control system according to (4) or (5), wherein the culture control information generation unit limits a type of the culture data used for generating culture control information.
(7) The cell culture control system according to any one of (1) to (6), wherein the culture control information generation unit determines a growth phase, a stationary phase, and a death phase of the cell based on the culture data, and generates the culture control information so as to increase a feed agent input ratio in an order of the growth phase, the stationary phase, and the death phase.
(8) The cell culture control system according to (7), wherein the culture control information generation unit detects a decrease in an increasing trend of viable cell density for the cell, and determines shift from the growth phase to the stationary phase.
(9) The cell culture control system according to (7) or (8), wherein the culture control information generation unit determines shift from the stationary phase to the death phase based on viable cell density for the cell and an elapsed time.
(10) The cell culture control system according to any one of (1) to (9), wherein a cell culture control device includes the culture control information generation device, and operates in an edge computing method.
(11) A cell culture control method causing
   a culture control information generation device to:
   acquire culture data obtained from a bioreactor that cultures a cell and an analyzer that analyzes a culture state of the cell; and
   generate culture control information based on the culture data, and causing
   a cell culture control device to:
      acquire the culture control information from the culture control information generation device; and
      perform, on the bioreactor, control of allowing the cell to grow or extending a lifespan of the cell in accordance with the culture control information.
(12) A cell culture control program causing
   a first computer to execute processing of:
   acquiring culture data obtained from a bioreactor that cultures a cell and an analyzer that analyzes a culture state of the cell; and
   generating culture control information based on the culture data, and causing
   a second computer to execute processing of:
      acquiring the culture control information from the first computer; and
      performing, on the bioreactor, control of allowing the cell to grow or extending a lifespan of the cell in accordance with the culture control information. Reference Signs List

1 BIOREACTOR CONTROL DEVICE
2 ADVICE GENERATION SERVER
3 BIOREACTOR
4 ANALYZER
11 TRANSMISSION/RECEPTION UNIT
12 INPUT/OUTPUT CONTROL UNIT
13 DATA CONVERSION UNIT
14 CONTROL UNIT
15 OPERATION SUPPORT UNIT
21 TRANSMISSION/RECEPTION UNIT
22 STORAGE
23 ADVICE GENERATION UNIT
24 LEARNING UNIT
100 CELL CULTURE CONTROL SYSTEM
151 ADVICE PROCESSING UNIT
152 ADVICE INPUT UNIT
153 DATA INPUT UNIT

## Claims

1. A cell culture control system comprising: a bioreactor that cultures a cell; an analyzer that analyzes a culture state of the cell; a cell culture control device; and a culture control information generation device,
wherein the culture control information generation device includes
a culture control information generation unit that generates culture control information based on culture data obtained from the bioreactor and the analyzer, and
the cell culture control device includes:
a control unit that controls the bioreactor; and
a culture control information processing unit that acquires the culture control information from the culture control information generation device, and instructs the control unit to perform, on the bioreactor, control of allowing the cell to grow or extending a lifespan of the cell in accordance with the culture control information.

2. The cell culture control system according to claim 1, wherein the culture control information processing unit confirms whether or not to accept the culture control information, and, when the culture control information is accepted, instructs the control unit to control the bioreactor in accordance with the culture control information.

3. The cell culture control system according to claim 1, wherein the control unit collects the culture data from the bioreactor and the analyzer, and transmits the culture data to the culture control information generation device.

4. The cell culture control system according to claim 3, wherein the culture control information processing unit causes the control unit to control the bioreactor so as to increase a frequency of collection of the culture data in a growth phase of a cell as compared with those in a stationary phase and a death phase.

5. The cell culture control system according to claim 4, wherein the culture control information processing unit causes the control unit to control the bioreactor so as to increase the frequency of collection of the culture data obtained from the bioreactor that is allowed to adjust the frequency of collection.

6. The cell culture control system according to claim 4, wherein the culture control information generation unit limits a type of the culture data used for generating culture control information.

7. The cell culture control system according to claim 1, wherein the culture control information generation unit determines a growth phase, a stationary phase, and a death phase of the cell based on the culture data, and generates the culture control information so as to increase a feed agent input ratio in an order of the growth phase, the stationary phase, and the death phase.

8. The cell culture control system according to claim 7, wherein the culture control information generation unit detects a decrease in an increasing trend of viable cell density for the cell, and determines shift from the growth phase to the stationary phase.

9. The cell culture control system according to claim 7, wherein the culture control information generation unit determines shift from the stationary phase to the death phase based on viable cell density for the cell and an elapsed time.

10. The cell culture control system according to claim 1, wherein a cell culture control device includes the culture control information generation device, and operates in an edge computing method.

11. A cell culture control method causing
a culture control information generation device to:
acquire culture data obtained from a bioreactor that cultures a cell and an analyzer that analyzes a culture state of the cell; and
generate culture control information based on the culture data, and causing
a cell culture control device to:
acquire the culture control information from the culture control information generation device; and
perform, on the bioreactor, control of allowing the cell to grow or extending a lifespan of the cell in accordance with the culture control information.

12. A cell culture control program causing
a first computer to execute processing of:
acquiring culture data obtained from a bioreactor that cultures a cell and an analyzer that analyzes a culture state of the cell; and
generating culture control information based on the culture data, and causing
a second computer to execute processing of:
acquiring the culture control information from the first computer; and
performing, on the bioreactor, control of allowing the cell to grow or extending a lifespan of the cell in accordance with the culture control information.
